# EUROPÄISCHE PATENTSCHRIFT

(11) **EP 0 509 547 B1**
(45) Veröffentlichungstag und Bekanntmachung des Hinweises auf die Patenterteilung: **02.02.2000**
(21) Anmeldenummer: 92106771.6
(22) Anmeldetag: 21.04.1992
(51) Int. Cl.: A61L 17/00, A61B 17/12

(54) **Chirurgisches Nahtmaterial**
Surgical suture
Suture chirurgicale

(30) Priorität: 19.04.1991 DE 4112909; 06.05.1991 DE 4114773
(43) Veröffentlichungstag der Anmeldung: 21.10.1992
(73) Patentinhaber: SERAG-WIESSNER GmbH & CO. KG, D-95112 Naila (DE)
(72) Erfinder: Gallus, Hubert, W-8674 Naila (DE)
(74) Vertreter: Klingseisen, Franz, Dipl.-Ing.

(56) Entgegenhaltungen:
- EP-A- 0 331 503
- DE-A- 2 900 265
- FR-A- 1 579 776
- US-A- 3 963 031

## Beschreibung

Die Erfindung betrifft ein chirurgisches Nahtmaterial in Form von geflochtenen Fäden.

Aus DE-A-29 00 265 ist eine Schlinge aus fadenförmigem Werkstoff zum Legieren von Gewächsen oder von Körperteilen oder zum Anschlingen von Fremdkörpern in Körperhohlräumen bekannt, die durch Ziehen an zumindest einem Ende des fadenförmigen Werkstoffs verkleinerbar ist, und einen zur Sicherung der Schlinge gegen Wiedervergrößerung festlegbaren Knoten aufweist. Die Schlinge kann durch Imprägnierung des Fadenmaterials versteift sein.

Aus EP-A-331 503 ist ein chirurgisches Nahtmaterial in Form von beschichteten, geflochtenen Fäden bekannt, wobei die Beschichtung aus Wachs, Paraffin oder Siliconöl zur Verbesserung der Gleitfähigkeit des Nahtmaterials dient, das auch resorbierbar sein kann.

Aus US-A-3, 963 ist ein geflochtenes chirurgisches Nahtmaterial bekannt, das abschnittsweise mit Schmiermittel, z.B. Polyester, Paraffin oder Wachs, imprägniert ist.

EP-A-0 490 143 beschreibt die Beschichtung von chirurgischem Nahtmaterial mittels Cyanacrylat an den Enden zum Erleichtern des Anbringens einer Nadel, insbesondere bei geflochtenen Fäden, die auch resorbierbar sein können.

In der Chirurgie wird die Roeder'sche Schlinge für Unterbindungen eingesetzt. Diese Schlinge wird mit einem speziellen Knoten, dem Roederknoten, aus einem Catgutfaden geknüpft. Sie wird im allgemeinen an einem Ende eines Rohrstabes angebracht, mit dem sie in den Körper eingeführt und um das zu unterbindende Gewebe gelegt werden kann. Die Roeder'sche Schlinge wird daraufhin zusammengezogen und das Gewebe unterbunden.

Der steife Charakter des Catgutfadens ist hierbei eine wichtige Voraussetzung, um die Roeder'sche Schlinge möglichst einfach und bequem in den Körper einführen und an das Gewebe anlegen zu können. Speziell in der endoskopischen Operationstechnik, bei der durch eine kleine Öffnung die chirurgischen Werkzeuge in den Körper eingeführt werden, ist eine steife Fadenschlinge notwendig.

Außerdem wird eine Schlinge verlangt, die statt aus Catgut aus einem synthetischen resorbierbaren Material hergestellt ist. Es gibt sowohl monofile Fäden als auch geflochtene Fäden aus diesem resorbierbaren Material, wobei die monofilen Fäden zwar die gewünschten steifen Eigenschaften besitzen, sich aber nicht für den Roederknoten eignen. Die geflochtenen Fäden sind dagegen für das Knüpfen der Schlinge geeignet, aber ihnen fehlt die notwendige Steifheit.

Der Erfindung liegt die Aufgabe zugrunde, ein chirurgisches Nahtmaterial, insbesondere aus resorbierbarem Material zur Verfügung zu stellen, das für eine Schlingenbildung geeignet ist und eine Unterbindung ermöglicht.

Diese Aufgabe wird erfindungsgemäß durch die Merkmale der Ansprüche gelöst. Dadurch, daß die geflochtenen, insbesondere resorbierbaren Fäden mit einem medizinisch unbedenklichen Mittel imprägniert oder getränkt und dadurch versteift werden, kann daraus eine Roeder'sche Schlinge geknüpft werden, die durch die Versteifungsbehandlung die nötige Steifheit zum Einführen in den Körper und zum Anlegen um das zu unterbindende Gewebe aufweist.

Die Erfindung wird anhand eines Ausführungsbeispiels näher erläutert.

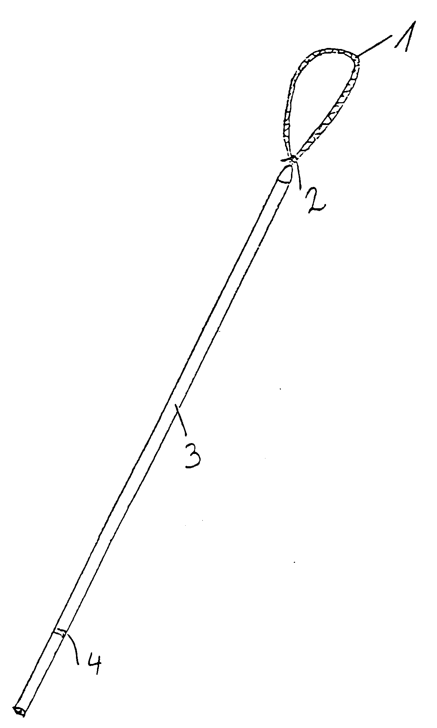

Die einzige Figur zeigt einen geflochtenen Faden 1 aus resorbierbarem, synthetischem Material, der mit einem Roederknoten 2 zu einer Schlinge von etwa 10 cm Länge geknüpft ist. Ein Ende dieser Schlinge ist durch einen Rohrstab 3 geführt, mittels dem die Schlinge in den Körper eingeführt wird.

Der an sich bekannte, geflochtene Faden 1 wird durch Tränken, Imprägnieren oder Beschichten mit einem im menschlichen Körper unschädlichen Material so behandelt, daß sich eine Versteifung des geflochtenen und zunächst sehr flexiblen Fadens ergibt. Die Versteifung des Fadens kann nach dem Knüpfen der Roederschlinge vorgenommen werden oder auch vorher, wenn dadurch das Knüpfen der Roederschlinge nicht beeinträchtigt wird. Es genügt, wenn nur der Bereich der Schlinge mit dem Versteifungsmaterial behandelt ist, nicht aber der im Rohrstab verlaufende Abschnitt des Fadens. Zur Erzielung der notwendigen Steifheit des geflochtenen Fadens kann in Abhängigkeit von dem verwendeten Versteifungsmittel jede geeignete Behandlung wie Tränken, Imprägnieren oder Beschichten des Fadenbündels vorgenommen werden.

Als Versteifungsmittel kommen alle Substanzen in Frage, die aus medizinischer Sicht unbedenklich sind und die während der Anwendung ihre steifmachende Wirkung nicht verlieren. Dazu können u.a. Pektine, Wachse und auch insbesondere jene Substanzen, die man in der Chirurgie als Klebstoff einsetzt, wie die verschiedenen Cyanacrylate verwendet werden. Ferner sind sogenannte biologische Klebstoffe, wie z.B. Gelatine-Resorzin-Gemische oder Fibrinogen-Thrombin-Gemische zum Versteifen geeignet. Ebenso kann Polyglykolsäure als Versteifungsmittel verwendet werden.

Vorzugsweise wird als Versteifungsmittel Cyanacrylat verwendet, das eine Viskosität von etwa 10 mPa hat und in Verbindung mit der Luftfeuchtigkeit polymerisiert. Hierdurch erhält man einen hohen Versteifungsgrad des Fadenmaterials. Cyanacrylat ist im Körper abbaubar und durch Verwendung von Cyanacrylat mit längeren Seitenketten kann eine toxische Wirkung vermieden werden. Dadurch, daß Cyanacrylat sehr flüssig aufgetragen werden kann, dringt es in geflochtene Fäden leicht ein, worauf nach Polymerisation durch Feuchtigkeitsaufnahme ein hoher Vernetzungsgrad und damit ein sehr standfester Faden erreicht wird, auch wenn ein zunächst sehr flexibler geflochtener resorbierbarer Faden verwendet wird. Ein derart starrer Faden wurde bisher in der Chirurgie nicht verwendet, vielmehr erhielt man bisher durch einen Oberflächenschutz des chirurgischen Nahtmaterials nur eine geringfügige Versteifung, die für die Bildung einer Roederschlinge mittels eines geflochtenen resorbierbaren Fadens nicht ausreicht.

Bei der Versteifungsbehandlung eines geflochtenen, sehr leicht biegsamen Fadens kann es ausreichend sein, nur einen Abschnitt zu versteifen, aus dem dann die Schlinge gebildet wird, während die weiterhin leicht biegsamen Abschnitte zum Knüpfen des Knotens und zum Einführen in den Rohrstab 3 verwendet werden.

Der Rohrstab 3 kann am hinteren Ende bei 4 mit einer Sollbruchstelle versehen sein. Der Faden ist dabei in das Endstück des Rohrstabes eingeklebt, so daß nach dem Abbrechen des Endstückes bei 4 die Schlinge mittels des Endstückes zugezogen werden kann.

Eine Versteifung des geflochtenen Nahtmaterials kann auch bei anderen als resorbierbaren Fäden von Vorteil sein. So ist bspw. ein geflochtenes Nahtmaterial ohne Versteifung dadurch mühsam zu verarbeiten, weil die geflochtenen Fäden an Organen kleben bleiben. Wird dagegen der geflochtene Faden mit einem Versteifungsmaterial behandelt und dadurch versteift, so wird dadurch die Verarbeitung des Fadens allgemein verbessert. Hierbei kann die Versteifung in einem geringeren Maß vorgenommen werden als bei einer Versteifung für die Ausbildung einer Roeder'schen Schlinge.

Ein derart versteifter Faden ist insbesondere auch für die Endoskopie und die Laparoskopie geeignet.

## Patentansprüche

1. Chirurgisches Nahtmaterial in Form von geflochtenen Fäden, insbesondere resorbierbaren Fäden, wobei die geflochtenen Fäden wenigstens abschnittsweise durch Pektine versteift sind.

2. Chirurgisches Nahtmaterial in Form von geflochtenen Fäden, insbesondere resorbierbaren Fäden, wobei die geflochtenen Fäden wenigstens abschnittsweise durch einen chirurgischen Klebstoff versteift sind.

3. Chirurgisches Nahtmaterial in Form von geflochtenen Fäden, insbesondere resorbierbaren Fäden, wobei die geflochtenen Fäden wenigstens abschnittsweise durch einen biologischen Klebstott versteift sind.

4. Chirurgisches Nahtmaterial in Form von geflochtenen Fäden, insbesondere resorbierbaren Fäden, wobei die geflochtenen Fäden wenigstens abschnittsweise, aber nicht ausschließlich am Ende durch Cyanacrylat versteift sind und einen hohen Versteifungsgrad aufweisen.

## Claims

1. Surgical suture in the form of braided fibers, especially resorbable fibers, wherein the braided fibers are at least in sections thereof stiffened by pectins.

2. Surgical suture in the form of braided fibers, especially resorbable fibers, wherein the braided fibers are at least in sections thereof stiffened by a surgical adhesive.

3. Surgical suture in the form of braided fibers, especially resorbable fibers, wherein the braided fibers are at least in sections thereof stiffened by a biological adhesive.

4. Surgical suture in the form of braided fibers, especially resorbable fibers, wherein the braided fibers are at least in sections thereof, but not exclusively at their ends, stiffened by cyanoacrylate and have a high degree of stiffening.

## Revendications

1. Suture chirurgicale avec des fils tressés, en particulier des fils résorbants, les fils tressés étant durcis au moins partiellement par de la pectine.

2. Suture chirurgicale avec des fils tressés, en particulier des fils résorbants, les fils tressés étant durcis au moins partiellement par une matière adhésive chirurgicale.

3. Suture chirurgicale avec des fils tressés, en particulier des fils résorbants, les fils tressés étant durcis au moins partiellement par une matière adhésive biologique.

4. Suture chirurgicale avec des fils tressés, en particulier des fils résorbants, les fils tressés étant durcis à l'extrémité au moins partiellement, mais pas exclusivement, par du cyanoacrylate possédant ainsi un degré de durcissement élevé.
